# EUROPEAN PATENT SPECIFICATION

(11) **EP 0 665 894 B1**
(45) Date of publication and mention of the grant of the patent: **19.11.2003**
(21) Application number: 93921888.9
(22) Date of filing: 30.09.1993
(51) Int. Cl.: C12Q 1/04, C12Q 1/10, C12N 1/20

(54) **CULTURE MEDIUM FOR THE IDENTIFICATION OF SALMONELLA AND PROCESS FOR ITS USE**
KULTURMEDIUM ZUM NACHWEIS SALMONELLA UND PROZESS ZUR VERWENDUNG DAVON
MILIEU DE CULTURE POUR L'IDENTIFICATION DE SALMONELLES ET SON PROCEDE D'UTILISATION

(30) Priority: 20.10.1992 FR 9212533
(43) Date of publication of application: 09.08.1995
(73) Proprietor: Rambach, Alain, F-75006 Paris (FR)
(72) Inventor: Rambach, Alain, F-75006 Paris (FR)
(74) Representative: Ahner, Francis
(86) International application number: EP9302659
(87) International publication number: WO94009152

(56) References cited:
- EUROPEAN JOURNAL OF MEDICAL CHEMISTRY vol. 25, no. 8 , 1990 , PARIS F pages 679 - 700 A. AGBAN ET AL. 'Synthèse de substrats indigogéniques. Mise en évidence de l activité estérastique des Salmonella.'

## Description

The present invention relates to an isolation medium permitting the identification of bacteria of the Salmonella species.

The identification of the bacterium Salmonella, which is pathogenic for man, is a major problem in medical bacteriology and in the monitoring of food hygiene.

Thus, in the case of epidemics transmitted by chicken farms, the birds infested in the intestinal tract are not ill but constitute a reservoir of Salmonella. The latter can be propagated, especially by eggs, in the diet following these epidemics. In fact, Salmonella is a bacterium whose notification is obligatory.

Nowadays, it is becoming essential to provide for the large scale detection of infected sites and more particularly of farms infected with the Salmonella bacterium in order to reduce these epidemics.

Indeed, Salmonella are generally to be recognised among other microorganisms such as the commensal species Escherichia coli and Proteus.

The detection of Salmonella is normally carried out on agar medium for the selective isolation of enterobacteria which permits the differentiation of pathogenic enterobacteria and the detection of suspicious Salmonella colonies. An ideal isolation medium should permit the growth of enterobacteria, the differentiation of the various species present so as to permit subsequent identification of a colony of each type, and the detection of suspicious Salmonella colonies.

The object of the present invention is to make it possible to carry out highly simplified routine tests for Salmonella, for example methods for monitoring the microbiological quality of water using soaking methods, or the monitoring of the microbiological quality of food factories using contact methods, the diagnosis or alternatively the detection of pathogenic microorganisms using traditional microbiological Petri dish methods on solid medium or on semi-solid selective medium, by means of media according to the invention.

The object of the invention is to permit the detection and differentiation of Salmonella by revealing an activity specific to these bacteria.

The Salmonella bacterium has among its range of enzymes esterases which cleave in particular caprylate derivatives such as nitrophenyl caprylate.

It could therefore be envisaged to use in an isolation medium a chromogen attached to the caprylate and its derivatives, a substrate for esterases, which would be liberated into the medium under the action of these enzymes, allowing the identification of Salmonella colonies.

However, tests on chromogens such as 5-bromo-4-chloro-3indolyl caprylate in a conventional culture medium for Salmonella have not made it possible to identify colorations specific for the inoculated colonies.

Some chromogenic compounds linked to fatty acids have been studied as substrates for *Salmonella* in a conventional medium (Agban & Coll., Eur. J. Med. Chem., 1990, 25, 697-699).

It has been shown, surprisingly, that the addition of a detergent to the culture medium at an appropriate concentration, and which is compatible with a high Salmonella growth, made it possible to obtain a coloration of the Salmonella colonies.

The present invention therefore relates to a culture medium with a chromogenic agent for the identification of Salmonella bacteria comprising a compound linked to a C₇-C₁₀ fatty acid and sodium deoxycholate, characterized in that the chromogenic agent results in the combination of the compound linked to a C₇-C₁₀ fatty acid and the sodium deoxycholate at a concentration greater than 1 g/l, preferably of between 1.5 g/l and 5 g/l.

Preferably, the chromogenic compound is chosen from caprylic acid esters, advantageously chosen from derivatives of indolyl caprylate and their salts, in particular 5-bromo-4-chloro-3 indolyl caprylate.

Preferably, the 5-bromo-4-chloro-3-indolyl caprylate will be used at a concentration of between 40 and 200 mg/l.

Advantageously, the chromogenic compound/appropriate detergent weight ratio is between 1/10 and 1/100.

It should be noted that the present invention does not consist in a mere addition of detergent to an identification medium, and that it is necessary to consider the chromogenic compound/appropriate detergent pair.

Thus, for the indolyl caprylate derivatives, only the fused polycyclic detergents, derivatives of cholane, make it possible to obtain a coloration of the Salmonella colonies, whereas its replacement with a detergent such as the standard anionic detergents (especially the detergents marketed under the trade mark TERGITOL by the company UNION CARBIDE Corp.) does not make it possible to identify a coloration.

It is also particularly important to note that the chromogenic agent of the medium according to the invention does not result in coloration with many other enterobacteria even if they are allowed to incubate for several weeks.

A person skilled in the art will of course know how to supplement the medium according to the present invention so as to eliminate exceptional non-Salmonella bacteria, especially by identifying other Salmonella-specific properties, in particular the properties relating to cleavage or metabolism of beta-galactosides and beta-glucosides.

Advantageously, the medium according to the present invention will also contain beta-glucosides and/or beta-galactosides.

Thus, a coloured Salmonella colony will be easily identified on a microbiological device even in the presence of thousands of other colonies, or on a semi-solid medium in order to increase the specificity of detection.

A deep blue coloration characteristic of the presence of Salmonella is generally obtained. However, other colorations may be envisaged depending on the chromogenic compound used.

Moreover, the detection will be facilitated by the fact that it necessitates neither the use of a non-viable coloured indicator nor that of a specific illumination.

The present invention also relates to a process for detecting strains of Salmonella in any sample, for which the culture medium as described above is inoculated with the said sample and in that the coloration characteristic of the presence of Salmonella is at least identified.

Depending on the form and presentation of the medium and depending on the test carried out, the medium is preferably inoculated by contact, by soaking, by surface inoculation or by inoculation within the mass.

Without further elaboration, it is believed that one skilled in the art can, using the preceding description, utilize the present invention to its fullest extent. The preferred specific embodiments are, therefore, to construed as merely illustrative, and not limitative of the disclosure in any way whatsoever.

The examples below are intended to illustrate other characteristics and advantages of the present invention.

### EXAMPLE: Detection of Salmonella strains

Various concentrations of sodium deoxycholate are added to the medium S (in g/l: meat extract 1; yeast extract 2; peptone 5; sodium chloride 5; agar 15; 5-bromo-4-chloro-3-indolyl caprylate 0.2).

The dishes are incubated at 37°C and the blue coloration of the colonies of the bacterium Salmonella AR3001 and of the control bacterium E. coli AR2901 is examined after incubating for 24 hours at 37°C.

Salmonella is identified only with the chromogenic substrate and deoxycholate combination.

| | Salmonella AR3001 | E. coli AR2901 |
|---|---|---|
| Deoxycholate 0.5 g/l | colourless | colourless |
| Deoxycholate 1.0 g/l | light blue | colourless |
| Deoxycholate 1.5 g/l | blue | colourless |
| Deoxycholate 2.0 g/l | darkish blue | colourless |
| Deoxycholate 2.5 g/l | dark blue | colourless |
| Deoxycholate 3.0 g/l | dark blue | colourless |

Various enterobacteria strains are isolated on the medium T (in g/l): meat extract 1; yeast extract 2; peptone 5; sodium chloride 5; agar 15; neutral red 0.03; Cellobiose 10; sodium deoxycholate 2.5; 5-bromo-4-chloro-3-indolyl caprylate 0.2).

The dishes are incubated at 37°C and the coloration of the colonies is examined after incubating for 24 hours.

The coloration of the colonies makes it possible to distinguish Salmonella from the other enterobacteria.

| | | |
|---|---|---|
| Salmonella | AR3001 | blue |
| Salmonella | AR3002 | blue |
| Salmonella | AR3003 | blue |
| Salmonella | AR3004 | blue |
| Salmonella | AR3004 | blue |
| Citrobacter | AR30033 | pink |
| Citrobacter | AR3437 | pink |
| Citrobacter | D9316 | pink |
| Citrobacter | D9352 | pink |
| Serratia | AR3493 | pink |
| Enterobacter | AR3494 | pink |
| Klebsiella | B2497 | pink |
| Klebsiella | B2593 | pink |

## Claims

1. Culture medium for the identification of Salmonella bacteria comprising a chromogenic compound linked to a C₇-C₁₀ fatty acid, and sodium deoxycholate, **characterized in that** sodium deoxycholate concentrations are between 1.5 g/l and 5 g/l.

2. Culture medium according to Claim 1, **characterized in that** the chromogenic compound is chosen from caprylic acid esters.

3. Culture medium according to Claim 2, **characterized in that** the chromogenic compound is chosen from derivatives of indolyl caprylate and their salts.

4. Culture medium according to Claim 3, **characterized in that** the chromogenic compound is 5-bromo-4-chloro-3-indolyl caprylate.

5. Culture medium according to Claim 4, **characterized in that** the 5-bromo-4-chloro-3-indolyl caprylate is used at a concentration of between 40 and 200 mg/l.

6. Culture medium according to one of Claim 1 to 5, **characterized in that** the chromogenic compound/sodium deoxycholate weight ratio is between 1/10 and 1/100.

7. Culture medium according to one of Claim 1 to 6, **characterized in that** it is presented in a liquid, semi-liquid or solid form.

8. Process for detecting Salmonella strains in any sample, **characterized in that** the culture medium according to any one of Claims 1 to 7 is inoculated with the said sample, and **in that** the coloration characteristic of the presence of Salmonella is identified.

9. Detection process according to Claim 8, **characterized in that** the culture medium is inoculated by contact, by soaking, by surface inoculation or deposition or inoculation within the mass.

## Patentansprüche

1. Kulturmedium für die Identifikation von Salmonella-Bakterien, umfassend eine chromogene Verbindung, die mit einer (C₇-C₁₀)-Fettsäure verknüpft ist, und Natriumdesoxycholat, **dadurch gekennzeichnet, dass** die Natriumdesoxycholat-Konzentrationen zwischen 1,5 g/l und 5 g/l liegen.

2. Kulturmedium nach Anspruch 1, **dadurch gekennzeichnet, dass** die chromogene Verbindung aus Caprylsäureestern ausgewählt ist.

3. Kulturmedium nach Anspruch 2, **dadurch gekennzeichnet, dass** die chromogene Verbindung aus Derivaten von Indolylcaprylat und deren Salzen ausgewählt ist.

4. Kulturmedium nach Anspruch 3, **dadurch gekennzeichnet, dass** die chromogene Verbindung 5-Brom-4-chlor-3-indolylcaprylat ist.

5. Kulturmedium nach Anspruch 4, **dadurch gekennzeichnet, dass** das 5-Brom-4-chlor-3-indolylcaprylat bei einer Konzentration zwischen 40 und 200 mg/l verwendet wird.

6. Kulturmedium nach einem der Ansprüche 1 bis 5, **dadurch gekennzeichnet, dass** das Gewichtsverhältnis chromogene Verbindung/Natriumdesoxycholat zwischen 1/10 und 1/100 liegt.

7. Kulturmedium nach einem der Ansprüche 1 bis 6, **dadurch gekennzeichnet, dass** es in flüssiger, halbflüssiger oder fester Form vorliegt.

8. Verfahren zum Nachweis von Salmonella-Stämmen in irgendeiner Probe, **dadurch gekennzeichnet, dass** das Kulturmedium nach irgendeinem der Ansprüche 1 bis 7 mit der Probe geimpft wird und dass die Färbungscharakteristik der Anwesenheit von Salmonella identifiziert wird.

9. Nachweisverfahren nach Anspruch 8, **dadurch gekennzeichnet, dass** das Kulturmedium durch Kontaktieren, durch Einweichen, durch Oberflächenbeimpfung oder -abscheidung oder durch Einimpfung innerhalb der Masse geimpft wird.

## Revendications

1. Milieu de culture pour l'identification de bactéries appartenant au genre *Salmonella*, comprenant un composé chromogène lié à un acide gras en C₇-C₁₀, et du désoxycholate de sodium, **caractérisé en ce que** les concentrations de désoxycholate de sodium sont comprises entre 1,5 g/l et 5 g/l.

2. Milieu de culture selon la revendication 1, **caractérisé en ce que** le composé chromogène est choisi parmi les esters d'acide caprylique.

3. Milieu de culture selon la revendication 2, **caractérisé en ce que** le composé chromogène est choisi parmi des dérivés de caprylate d'indolyle et leurs sels.

4. Milieu de culture selon la revendication 3, **caractérisé en ce que** le composé chromogène est le caprylate de 5-bromo-4-chloro-3-indolyle.

5. Milieu de culture selon la revendication 4, **caractérisé en ce que** le caprylate de 5-bromo-4-chloro-3-indolyle est utilisé à une concentration comprise entre 40 et 200 mg/l.

6. Milieu de culture selon l'une quelconque des revendications 1 à 5, **caractérisé en ce que** le rapport pondéral composé chromogène/désoxycholate de sodium est compris entre 1:10 et 1:100.

7. Milieu de culture selon l'une quelconque des revendications 1 à 6, **caractérisé en ce qu'**il est présenté sous une forme liquide, semi-liquide ou solide.

8. Procédé pour la détection de souches de *Salmonella* dans un échantillon quelconque, **caractérisé en ce que** le milieu de culture selon l'une quelconque des revendications 1 à 7 est ensemencé avec ledit échantillon, et **en ce que** la coloration caractéristique de la présence de *Salmonella* est identifiée.

9. Procédé de détection selon la revendication 8, **caractérisé en ce que** le milieu de culture est ensemencé par contact, par immersion, par inoculation en surface ou dépôt ou inoculation dans la masse.
